# EUROPEAN PATENT SPECIFICATION

(11) **EP 2 537 528 B1**
(45) Date of publication and mention of the grant of the patent: **16.09.2015**
(21) Application number: 12172907.3
(22) Date of filing: 21.06.2012
(51) Int. Cl.: A61K 38/47, C12N 9/38, C12N 9/40, A61P 1/04

(54) **Pharmaceutical compositions containing beta- and alpha-galactosidase and their use in the treatment of gastroesophageal reflux disease**
Pharmazeutische Zusammensetzungen enthaltend Beta- und Alpha-Galactosidase und ihre Verwendung in der Behandlung der Refluxösophagitis
Compositions pharmaceutiques contenant de la beta- et alpha-galactosidase et leur utilisation dans le traitement du reflux gastro-oesophagien

(30) Priority: 24.06.2011 IT MI20111154
(43) Date of publication of application: 26.12.2012
(73) Proprietor: ALFA WASSERMANN S.p.A., 65020 Alanno (PE) (IT)
(72) Inventor: Zanarotti, Alessandro, 20129 MILANO (IT); Brunetti, Gabriele, 20129 MILANO (IT); Cecchetti, Sergio, 20060 POZZUOLO MARTESANA (MI) (IT)
(74) Representative: Minoja, Fabrizio

(56) References cited:
- EP-A2- 0 176 971
- WO-A1-90/14101
- DE-U1-202006 012 340
- GROSSI L ET AL: "EFFECT OF ALPHA-GALACTOSIDASE ON 24-HOUR ESOPHAGEAL pH PROFILE IN PATIENTS WITH GASTRO-ESOPHAGEAL REFLUX DISEASE", DIGESTIVE AND LIVER DISEASE, W.B. SAUNDERS, GB, vol. 41, 1 March 2009 (2009-03-01), page S49, XP026191164, ISSN: 1590-8658, DOI: 10.1016/S1590-8658(09)60126-2 [retrieved on 2009-03-01]
- GROSSI L ET AL: "M1881 Effect of Alpha-Galactosidase On 24-Hour Esophageal pH Profile in Patiens with Gastro-Esophageal Reflux Disease", GASTROENTEROLOGY, ELSEVIER, PHILADELPHIA, PA, vol. 136, no. 5, 1 May 2009 (2009-05-01), pages A-438, XP026112563, ISSN: 0016-5085, DOI: 10.1016/S0016-5085(09)62016-3 [retrieved on 2009-05-01]

## Description

### Field of invention

The present invention relates to pharmaceutical or nutritional compositions containing α-galactosidase and β-galactosidase. Said compositions are useful in the prevention and/or treatment of gastroesophageal reflux disease.

### State of the art

Gastroesophageal reflux disease (GERD), also known as gastro-oesophageal reflux disease (GORD), arises when the stomach contents flow back into the oesophagus.

It is an extremely common disorder, characterised by heartburn and regurgitation.

Reflux of the stomach contents into the oesophagus is a physiological phenomenon; when the frequency of the episodes increases, the reflux becomes harmful to the oesophageal mucosa and causes the appearance of symptoms and complications, such as oesophagitis and Barrett's oesophagus.

As the most harmful constituent of reflux is hydrochloric acid, the treatment is based on suppression of the acid secretion.

The treatment of GERD greatly improved with the advent of proton pump inhibitors (PPIs), which lead to (endoscopic) healing of oesophagitis in over 90% of cases.

PPIs therefore represent the first-choice treatment in GERD.

However, although PPIs reduce the acidity of the gastric juices, the number of reflux episodes remains unchanged, so the oesophageal mucosa is still exposed to the action of the gastric juices.

H2 antihistamines (anti-H2s) and antacids are also used to treat the symptoms of gastroesophageal reflux disease.

Despite the availability of various therapeutic approaches, the success rates are much lower if remission and improvement of symptoms are also considered.

Up to 40% of GERD patients have poorly controlled symptoms, which indicates that there is still a need for new therapeutic agents to treat the disorder.

Moreover, there is known to be a relationship between diet and reflux. For example, it is known that a meal rich in fats increases the symptoms of reflux and the frequency of reflux episodes by reducing the pressure of the lower oesophageal sphincter. The relationship between other constituents of the diet and reflux is less well known.

It has been demonstrated that the administration of lactose to volunteers increases acid reflux episodes and markedly influences the function of the lower oesophageal sphincter. The effects of lactose are mediated by the production of short-chain fatty acids, which derive from the metabolism of lactose by the bacterial flora of the colon (Piche et al., 2000; Am J Physiol Gastrointest Liver Physiol 278: G578-84).

The administration of fructo-oligosaccharides (FOS) also induces effects on the pressure of the lower oesophageal sphincter and the pH of the oesophageal lumen similar to those observed with lactose administration (Piche et al., Gastroenterology 2003; 124: 894-902).

These data suggest that fermentation in the colon of wholly or partly unabsorbed carbohydrates may be responsible for the onset or aggravation of gastroesophageal reflux disease.

A preliminary study (9 cases - methodologically elegant and placebo-controlled) has demonstrated that a preparation based on alpha-galactosidase acts as reflux inhibitor (Grossi et al., Digestive and Liver Disease 41S: FISMAD Milan, 28/3-1/4 2009; S49 - Abstract).

In that study, the administration of alpha-galactosidase for 4 weeks reduced the frequency of Transient Lower Oesophageal Sphincter Relaxations (TLES relaxation), reflux episodes, the percentage of pH < 4 and the severity of the reflux symptoms.

As the disorder is so widespread, there is still a need to use alternative compositions for the prevention and/or treatment of gastroesophageal reflux disease.

### Summary of the invention

The present invention relates to pharmaceutical or nutritional compositions containing α-galactosidase and β-galactosidase.

The invention also relates to the use of said compositions in the prevention and/or treatment of gastroesophageal reflux disease.

### Description of the invention

The present invention relates to pharmaceutical or nutritional compositions containing α-galactosidase and β-galactosidase as active ingredients.

It has now surprisingly been found that pharmaceutical or nutritional compositions containing α-galactosidase and β-galactosidase are useful in the prevention and/or treatment of gastroesophageal reflux disease (GERD). Said compositions have a different action mechanism from those of the medicaments currently used (acid secretion inhibitors) in that they inhibit reflux or at least reduce its frequency.

The present invention represents a new therapeutic strategy, which is designed in particular for non-responders and patients with a poor response to the current treatments based on proton pump inhibitors.

The pharmaceutical or nutritional compositions to which the present invention relates can be also added to the existing treatments (as an "add-on therapy"), leading to a significant improvement in the quality of life of many patients with gastroesophageal reflux disease.

The present invention relates to pharmaceutical or nutritional compositions containing a combination of the active ingredients, mixed with at least one pharmaceutically acceptable excipient or carrier.

Said compositions are preferably administered orally.

The pharmaceutical or nutritional compositions for the prevention and/or treatment of gastroesophageal reflux disease containing α-galactosidase and β-galactosidase as active ingredients can be formulated by conventional methods.

The preferable dosage forms are solid formulations for oral administration, such as powders, granules, tablets, pills and capsules.

When these compositions are prepared, the active ingredients can be mixed with at least one pharmaceutically acceptable excipient or carrier, such as an inert diluent, a lubricant such as magnesium stearate, an anti-caking agent such silicon dioxide, a preservative, an antioxidant, a disintegrant, a binder, a thickening agent, a buffer, a sweetener such as acesulfame potassium, a flavouring agent, etc..

Further dosage forms can be liquid formulations for oral administration including, for example, emulsions, syrups, elixirs, suspensions and solutions.

The dose of the active ingredients for each patient is determined on the basis of age, body weight, general state of health, gender, diet, condition of the disease treated and/or other factors.

The quantity of the active ingredients in the composition can range from 75 GalU to 2400 GalU for α-galactosidase, preferably from 300 GalU to 1200 GalU, and most preferably amounts to 300 GalU in the form of dosage units, and from 2500 to 12500 LactU for β-galactosidase, preferably from 3500 to 11000 LactU, and most preferably amounts to 4500 LactU in the form of dosage units.

The dosage unit form is usually administered one or more times a day, as required.

α-Galactosidase has proved to prevent the onset of bloating and flatulence symptoms after intake of Non-digestible Oligosaccharides (NDOs) and to reduce the intensity of heartburn and the frequency of regurgitation episodes during repeated administration to patients with gastroesophageal reflux disease.

The combination of α-galactosidase and β-galactosidase has provided an unexpected symptomatic benefit in some patients, who respond unsatisfactory to conventional therapy based on PPI. There is initial evidence that the combination of the two substances boosts the effects by reducing the frequency and duration of reflux to an extent which is markedly high.

The examples given below further illustrate the invention.

### Examples

### Formulation example 1

The composition according to the invention in the form of a tablet to be swallowed or chewed contains:
69 mg β-galactosidase (65000 LactU/g)
30 mg α-galactosidase (10000 GalU/g)
280 mg sorbitol
22.78 mg flavouring
8 mg crosslinked sodium carboxymethylcellulose
8 mg magnesium stearate
2 mg silicon dioxide
0.22 mg acesulfame potassium

### Formulation example 2

The composition according to the invention in the form of a tablet to be swallowed contains:
69 mg β-galactosidase (65000 LactU/g)
30 mg α-galactosidase (10000 GalU/g)
280 mg sorbitol
8 mg crosslinked sodium carboxymethylcellulose
8 mg magnesium stearate
2 mg silicon dioxide
30 mg ginger (Zingiber officinale) extract

## Claims

1. Pharmaceutical or nutritional compositions containing α-galactosidase and β-galactosidase, wherein the content of α-galactosidase ranges from 75 and 2400 GaIU and the content of β-galactosidase ranges from 2500 to 12500 LactU, in the form of a dosage unit and at least one pharmaceutically acceptable excipients or carrier.

2. Pharmaceutical or nutritional composition according to claim 1 for use in the prevention and /or treatment of gastroesophageal reflux disease.

## Patentansprüche

1. Pharmazeutische Zusammensetzung oder Nahrungszusammensetzung, enthaltend α- Galaktosidase und β- Galaktosidase, worin der Gehalt an α- Galaktosidase von 75 bis 2400 GaIU reicht und der Gehalt an β-Galaktosidase von 2500 bis 12500 LactU reicht, in der Form einer Dosiereinheit und mindestens einen pharmazeutisch annehmbaren Exzipienten oder Träger.

2. Pharmazeutische Zusammensetzung oder Nahrungszusammensetzung nach Anspruch 1 für die Verwendung in der Prävention und/oder Behandlung der gastroösophagealen Refluxkrankheit.

## Revendications

1. Compositions pharmaceutiques ou nutritionnelles contenant une α-galactosidase et une β-galactosidase, dans lesquelles la teneur en α-galactosidase est comprise entre 75 et 2 400 UGal et la teneur en β-galactosidase est comprise entre 2 500 et 12 500 ULact, sous la forme d'une unité posologique et au moins un excipient ou un support pharmaceutiquement acceptable.

2. Composition pharmaceutique ou nutritionnelle selon la revendication 1, destinée à être utilisée dans la prévention et/ou le traitement du reflux gastro-oesophagien pathologique.
